# EUROPÄISCHE PATENTANMELDUNG

(11) **EP 3 438 688 A1**
(43) Veröffentlichungstag der Anmeldung: **06.02.2019**
(21) Anmeldenummer: 17184518.3
(22) Anmeldetag: 02.08.2017
(51) Int. Cl.: G01R 33/54, G01R 33/567

(54) **AUTOMATISCHE AUSWAHL DER BILDGEBUNGSSEQUENZ UNTER BERÜCKSICHTIGUNG VON ATEMDATEN**

(71) Anmelder: Siemens Healthcare GmbH, 91052 Erlangen (DE)
(72) Erfinder: Thörmer, Gregor, 90556 Cadolzburg (DE)

(57) **Zusammenfassung**

Die Erfindung betrifft ein Verfahren zur automatischen Aufnahme von MR-Bildern, wobei eine Atembewegung der Untersuchungsperson durch einen an der MR-Anlage angebrachten Atemsensor detektiert wird. Weiterhin wird anhand der detektierten Atembewegung eine Atemunterbrechung detektiert sowie eine Dauer der Atemunterbrechung. Es wird automatisch eine Bildgebungssequenz zur Erstellung von MR-Bildern unter Berücksichtigung der detektierten Dauer der Atemunterbrechung ausgewählt.

## Beschreibung

Die vorliegende Erfindung betrifft ein Verfahren zur Aufnahme von MR-Bildern einer Untersuchungsperson in einer MR-Anlage und die zugehörige MR-Anlage hierfür. Die Erfindung betrifft weiterhin ein Computerprogrammprodukt und einen elektronisch lesbaren Datenträger mit darauf gespeicherten elektronisch lesbaren Steuerinformationen.

Zur Vermeidung von Bewegungsartefakten bei der Untersuchung einer Untersuchungsperson, insbesondere des Abdomens, werden MR-Aufnahmen zum großen Teil mit der Atemanhalte-Technik durchgeführt, bei der die Untersuchungsperson ein- oder mehrmals die Luft anhält bzw. die Atmung unterbricht während der Aufnahme der MR-Signale. Je länger die Untersuchungsperson hierfür die Luft am Stück anhalten kann, desto schneller können alle notwendigen MR-Daten aufgenommen werden. Es sind jedoch auch alternative Aufnahmetechniken bekannt, wie beispielsweise radiale Aufnahmetechniken und Techniken, die unter dem Begriff "Star Vibe", "Grasp Vibe" bekannt sind, verbreitet, die eine Aufnahme der MR-Signale bei freier Atmung erlauben. Etwa 20% aller Untersuchungspersonen können nicht oder nur inadäquat die Luft anhalten und sind somit nur für eine Untersuchungstechnik geeignet, bei der sie durchgehend atmen können. Aufgrund der besseren Qualität sind jedoch für die übrigen Untersuchungspersonen die Bildgebungssequenzen mit Atemanhalte-Technik zu bevorzugen.

Insofern ist die Auswahl der optimalen Untersuchungsstrategie und der verwendeten Bildgebungssequenz mit der Wahl der Tatsache, ob eine Atemanhalte-Technik oder eine freie Atmung verwendet wird, und die Festlegung einer maximalen Anhalte-Kapazität von zentraler Bedeutung, die eine Bedienperson für jeden untersuchten Patienten bzw. jede Untersuchungsperson individuell treffen muss. Dies geschieht oft unter Zeitdruck und in Unkenntnis der genauen physiologischen Möglichkeiten der Untersuchungsperson. Werden bei dieser Auswahl die falschen Entscheidungen getroffen, beispielsweise bei Auswahl der Untersuchung während der Atemanhalte-Technik, ist es im schlechtesten Fall möglich, dass eine Untersuchung wiederholt werden muss, wenn die Untersuchungsperson nicht in der Lage war, die notwendige Zeitdauer die Luft anzuhalten. Die Auswahl der Aufnahmetechnik mit oder ohne Atemanhalten wird üblicherweise auf Basis von Erfahrungswerten durch die Bedienperson durchgeführt. Beispielsweise kann die Bedienperson bei einem älteren übergewichtigen Patienten oder einem Patienten mit schlechtem Gesundheitszustand abschätzen, dass eine Atemunterbrechung von mehr als 10 sec pro Unterbrechung wahrscheinlich nicht möglich sein wird. Die Bedienperson muss daraufhin die Messung so einstellen, dass die Signalaufnahme während dieser abgeschätzten Zeitspanne erfolgt. Stimmt diese Schätzung nicht, sind die erzeugten MR-Bilder meist unbrauchbar und müssen mit kürzerer Atemanhalte-Dauer oder in freier Atmung wiederholt werden. Dies bedeutet, dass die gesamte MR-Untersuchung stark von der Erfahrung der Bedienperson abhängt, wobei trotzdem bei erfahrenen Bedienpersonen der Fall auftritt, dass Aufnahmen doch nicht - wie ursprünglich abgeschätzt - mit der Atemanhalte-Technik durchgeführt werden können.

DE 10 2014 220 460 A1 und DE 10 2012 217 262 DE beschreiben jeweils eine Anpassung von Parametern einer Pulssequenz.

Es besteht daher eine Aufgabe der vorliegenden Erfindung darin, MR-Aufnahmen so aufzunehmen, dass der Anteil der aufgenommenen MR-Daten, die durch Atmung unbrauchbar sind, minimiert wird und gleichzeitig eine bestmögliche Qualität der aufgenommenen MR-Bilder erreicht wird, unabhängig von der Erfahrung der Bedienperson der MR-Anlage.

Diese Aufgabe wird durch die Merkmale der unabhängigen Ansprüche gelöst. In den abhängigen Ansprüchen sind weitere Ausführungsformen beschrieben.

Gemäß einem ersten Aspekt wird ein Verfahren zur Aufnahme von MR-Bildern einer Untersuchungsperson in einer MR-Anlage bereitgestellt, das folgende automatisch ablaufende Schritte aufweist: Es erfolgt eine automatische Detektion einer Atembewegung der Untersuchungsperson durch einen an der MR-Anlage angebrachten Atemsensor. Weiterhin wird eine Atemunterbrechung bei der Untersuchungsperson detektiert und es wird automatisch die Dauer der Atemunterbrechung anhand der detektierten Atembewegung bestimmt. Anschließend wird automatisch die Bildgebungssequenz zur Erstellung der MR-Bilder unter Berücksichtigung der detektierten Dauer der Atemunterbrechung ausgewählt.

Durch den an der MR-Anlage angebrachten Atemsensor ist es möglich, automatisch die Atembewegung zu detektieren und es kann überprüft werden, wie lange die untersuchte Person die Luft anhalten kann. In Abhängigkeit von diesem Ergebnis kann die MR-Anlage dann automatisch die Bildgebungssequenz derart wählen, dass die Fähigkeit des Atemanhaltens berücksichtigt wird. Falls detektiert wird, dass die Untersuchungsperson den Atem nicht hinreichend anhalten kann, ist es beispielsweise möglich, die Bildgebungssequenz derart anzupassen, dass die Signalaufnahme noch in dem Zeitfenster aufgenommen werden kann, das durch die Atemanhalte-Dauer definiert ist. Ist dies nicht möglich, kann das System automatisch eine Bildgebungssequenz wählen, die ohne Atemanhalte-Technik brauchbare Informationen für den befundenden Arzt liefert.

Es ist möglich, dass eine Bildgebungssequenz ausgewählt wird, für die eine Atemunterbrechung mit einer ersten Dauer notwendig ist, wenn die detektiert Atemunterbrechung vorher größer als diese oder gleich dieser ersten Dauer ist. Wenn jedoch detektiert wird, dass die Dauer der möglichen Atemunterbrechung kleiner als ein vorbestimmter unterer Grenzwert ist, so wird eine Bildgebungssequenz automatisch ausgewählt, die ohne Atemunterbrechung aufgenommen werden kann und noch brauchbare, auswertbare Bildinformationen liefert. Wenn die Bildgebungssequenz und die zugehörigen Bildgebungsparameter nicht mehr so abgeändert werden können, dass MR-Bilder mit gewünschter Auflösung und Qualität von dem zu untersuchenden Bereich in der Atemanhalte-Technik aufgenommen werden können, so wählt die MR-Anlage automatisch eine Bildgebungssequenz aus, die ohne Atemanhalte-Technik arbeitet.

Eine Atemunterbrechung kann hierbei detektiert werden, wenn in einem zeitlichen Verlauf der Atembewegung ein Plateau detektiert wird, wobei die Dauer der Atemunterbrechung anhand der Länge des Plateaus im zeitlichen Verlauf bestimmt wird. Bei einer kontinuierlichen Atmung ergibt sich ein annähender sinusförmiger Verlauf des detektierten Signals, wobei das detektierte Signal aus der Änderung des Gütefaktors einer kleinen Empfangsspule, die in einem anderen Frequenzbereich sendet und empfängt als das MR-Anlage, abgeleitet wird. Diese Änderung ergibt sich in Abhängigkeit der Gewebezusammensetzung im Empfangsbereich der Spule, z.B. zeitlich veränderlicher Anteil von Lunge und Leber. Wenn nun nach dem Einatmen oder nach dem Ausatmen die Luft angehalten wird, ergibt sich ein im Wesentlichen gleichbleibendes Signal, das im zeitlichen Verlauf ein Plateau darstellt. Die Länge des Plateaus gibt dann die Dauer der Atemunterbrechung an.

Der Atemsensor kann beispielsweise in eine MR-Empfangsspule, wie beispielsweise die Rückenspule, integriert sein, die zum Empfang der MR-Signale verwendet wird. Ebenso ist es möglich, dass der Atemsensor direkt in dem Liegetisch integriert ist, auf dem die Untersuchungsperson liegt.

Bei dem Verfahren wird detektiert, ob die Untersuchungsperson in der MR-Anlage in der Aufnahmeposition liegt, bei der die MR-Bilder aufgenommen werden. Wenn die Untersuchungsperson nun in dieser endgültigen Position innerhalb der MR-Anlage liegt, so kann automatisch von der MR-Anlage ein gespeichertes Sprachkommando ausgegeben werden, das beispielsweise in einer Sprachdatei gespeichert ist, mit der die Untersuchungsperson aufgefordert wird, die Luft anzuhalten, entweder über einen bestimmten Zeitraum oder solange wie möglich.

Vorzugsweise wird zumindest ein Bildgebungsparameter der Bildgebungssequenz für die Aufnahme der MR-Bilder derart angepasst, dass die für die Bildgebungssequenz notwendige Atemunterbrechung kleiner als die detektieret Dauer der Atemunterbrechung ist. Bei dieser Ausführungsform wird soweit wie möglich die Bildgebungssequenz an die mögliche Atemanhalte-Dauer angepasst. In einer anderen Ausführungsform wird überprüft, ob eine notwendige Atemunterbrechung für eine Bildgebungssequenz durch die Untersuchungsperson erreicht wird oder nicht, und wenn dies nicht der Fall ist, wir direkt auf eine Bildgebungssequenz ohne Atemunterbrechung ausgewichen.

Ein Plateau in dem zeitlichen Verlauf kann beispielsweise identifiziert werden, indem Zeitabschnitte des Ausgangssignals des Atemsensors miteinander verglichen werden, wobei dann die Änderung des Ausgangssignals in konsekutiven Zeitabschnitte kleiner als ein Grenzwert ist. Diese Zeitabschnitte können überlappend sein oder nicht.

Die Erfindung betrifft weiterhin die hierfür ausgebildete MR-Anlage, die einen Atemsensor aufweist, der ausgebildet ist, automatisch die Atembewegung der Untersuchungsperson zu detektieren. Weiterhin ist eine Steuereinheit vorgesehen, die wie oben ausgeführt ausgebildet ist, um die Dauer der Atemunterbrechung anhand der detektierten Atembewegung zu detektieren und automatische eine Bildgebungssequenz zur Erstellung der MR-Bilder unter Berücksichtigung der detektierten Dauer der Atemunterbrechung auszuwählen.

Weiterhin ist ein Computerprogrammprodukt vorgesehen mit Programmmitteln, die direkt in eine Speichereinheit einer programmierbaren Steuereinheit der MR-Anlage ladbar sind. Um die Schritte des Verfahrens wie oben beschrieben auszuführen, werden die Programmmittel in der Steuereinheit ausgeführt.

Ebenso ist ein elektronisch lesbarer Datenträger mit darauf gespeicherten elektronisch lesbaren Steuerinformationen vorgesehen, wobei diese derart ausgestaltet sind, dass sie bei Verwendung des Datenträgers in einer Steuereinheit der MR-Anlage das Verfahren wie oben beschrieben durchführen.

Die Erfindung wird nachfolgend unter Bezugnahme auf die beiliegenden Zeichnungen näher erläutert.
- Fig. 1: zeigt schematisch eine MR-Anlage, die erfindungsgemäß überprüft, wie lange eine Untersuchungsperson den Atem anhalten kann und dann automatisch eine Bildgebungssequenz wählt;
- Fig. 2: zeigt schematisch den Verlauf eines Atemsignals bei durchgängiger Atmung und bei einer Atemunterbrechung;
- Fig. 3: zeigt schematisch ein Flussdiagramm mit den Schritten, die von der MR-Anlage ausgeführt werden zur automatischen Auswahl der Bildgebungssequenz.

Nachfolgend wird die vorliegende Erfindung anhand bevorzugter Ausführungsformen unter Bezugnahme auf die beiliegenden Zeichnungen näher erläutert. Die Figuren sind schematische Darstellungen verschiedener Ausführungsformen, wobei die in den Figuren dargestellten Elemente nicht notwendigerweise maßstabsgetreu dargestellt sind. Vielmehr sind die in den Figuren dargestellten Elemente derart wiedergegeben, dass die Funktionen und der Zweck für den Fachmann verständlich werden. Die in den Figuren dargestellten Verbindungen zwischen funktionellen Einheiten oder sonstigen Elementen können auch als indirekte Verbindungen implementiert werden, wobei eine Verbindung drahtlos oder drahtgebunden sein kann. Funktionelle Einheiten können als Hardware, Software oder eine Kombination aus Hard- und Software implementiert sein.

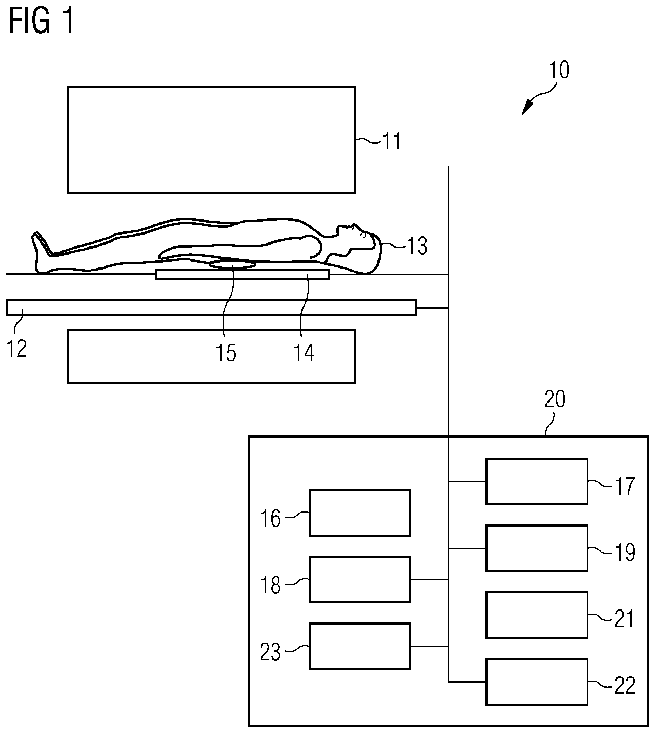

In Bezug auf Fig. 1 wird eine MR-Anlage 10 erläutert, mit der, wie nachfolgend erläutert wird, MR-Bildgebungssequenzen in Abhängigkeit von der Fähigkeit der Untersuchungsperson ausgewählt werden, wie lange diese den Atem anhalten kann. Die MR-Anlage 10 weist einen Magneten 11 zur Erzeugung eines Polarisationsfeldes B0 auf, wobei eine auf einer Liege 12 angeordnete Untersuchungsperson 13 in den Magneten gefahren wird, um dort ortscodierte Magnetresonanzsignale aus der Untersuchungsperson aufzunehmen. Weiterhin ist schematisch eine MR-Sende- und oder -Empfangsspule 14 dargestellt, hier eine Rückenspule, die insbesondere zur Signalaufnahme verwendet werden kann. Die Erfindung kann auch bei s.g. paralleler Bildgebung Anwendung finden, bei der die MR-Signale gleichzeitig mit mehreren nicht gezeigten Lokalspulen, einem Spulenarray, aufgenommen werden. Weiterhin ist ein Atemsensor 15 dargestellt, der in die MR-Spule, hier der Rückenspule, integriert ist. Der Atemsensor 15 kann weiterhin in der Liege 12 integriert sein, auf der die Untersuchungsperson liegt. Durch das Einstrahlen von Hochfrequenzpulsen und Schalten von Magnetfeldgradienten kann die durch das Polarisationsfeld B0 erzeugte Magnetisierung aus der Gleichgewichtslage ausgelenkt und ortscodiert werden, und die sich ergebende Magnetisierung wird von der oder den Empfangsspulen detektiert. Wie durch Einstrahlen von HF-Pulsen und durch Schalten von Magnetfeldgradienten in verschiedenen Kombinationen und Reihenfolgen MR-Bilder erzeugt werden können, ist dem Fachmann grundsätzlich bekannt und wird hier nicht näher erläutert.

Die MR-Anlage weist weiterhin eine Steuereinheit 20 auf, die zur Steuerung der MR-Anlage 10 verwendet werden kann. Die Steuereinheit 20 weist eine HF-Steuereinheit 16 für die Steuerung und Generierung der HF-Pulse zur Auslenkung der Magnetisierung auf. Eine Gradientensteuerung 17 ist für die Steuerung und Schaltung der notwendigen Gradienten vorgesehen. Eine Bildsequenzsteuerung 18 steuert die Abfolge der Magnetfeldgradienten, der Signaldetektion, der HF-Pulse und damit indirekt die Gradientensteuereinheit 17 und die HF-Steuereinheit 16. In der Bildsequenzsteuerung wird festgelegt, welche Gradientenstärken, welches Gesichtsfeld und welche Ortsauflösung verwendeten werden. Über eine Eingabeeinheit 19 kann eine Bedienperson die MR-Anlage steuern und auf einer Anzeige 23 können MR-Bilder und sonstige zur Steuerung notwendigen Informationen angezeigt werden. Eine Recheneinheit 21 mit mindestens einer Prozessoreinheit ist vorgesehen zur Steuerung der verschiedenen Einheiten in der Steuereinheit 20. Weiterhin ist eine Speichereinheit 22 vorgesehen, in der beispielsweise Programmmodule bzw. Programme abgespeichert sein können, die, wenn sie von der Recheneinheit 21 bzw. ihrer Prozessoreinheit ausgeführt werden, den Ablauf der MR-Anlage steuern können. Die gespeicherten Programme sind derart ausgebildet, dass sie bei Ausführung in der Recheneinheit automatisch die Atembewegung der Untersuchungsperson, die von dem Atemsensor 15 detektiert wird, untersuchen und insbesondere die Fähigkeit überprüfen, wie lange die Untersuchungsperson die Atmung anhalten kann.

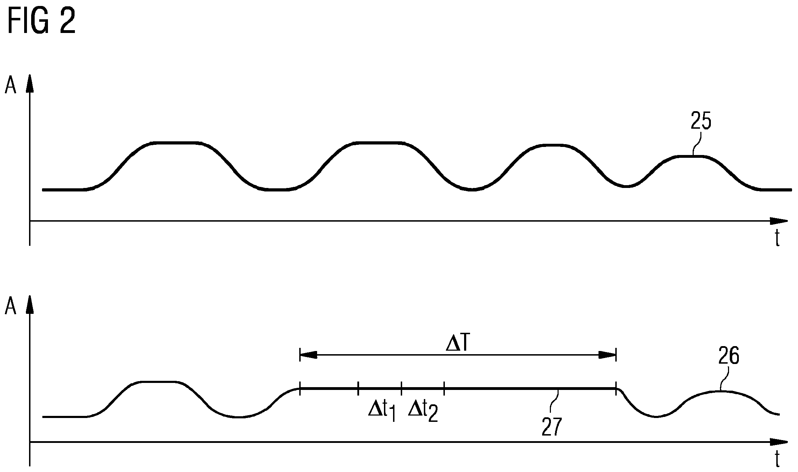

In Fig. 2 ist schematisch ein zeitlicher Verlauf 25 eines Signals dargestellt, wie es durch den Atemsensor 15 detektiert wird. Der Atemsensor kann so ausgebildet sein, dass er Informationen über die Atembewegungen der Untersuchungsperson aufzeichnet, sobald die Untersuchungsperson auf der Liege liegt. Somit ist es möglich, mit dieser Information vollautomatisch die Atemanhalte-Fähigkeit einer Untersuchungsperson noch vor der eigentlichen Untersuchung abzuschätzen und die Untersuchungsperson automatisch der optimalen Untersuchungsstrategie zuzuführen. Wenn die Untersuchungsperson an ihrer endgültigen Position in der MR-Anlage liegt, kann eine Funktionalität eines automatischen Atemkommandos genutzt werden. Hierbei weist die MR-Anlage beispielsweise ein automatisiertes Sprachkommando auf, das beispielsweise in einer Sprachdatei gespeichert sein kann. Dieses Sprachkommando kann ausgegeben werden, wenn detektiert wird, dass die Untersuchungsperson in ihrer endgültigen Position in der MR-Anlage liegt, wobei die Untersuchungsperson aufgefordert wird, die Luft anzuhalten. Ein mögliches Sprachkommando kann beispielsweise lauten: "Einatmen, Ausatmen, Einatmen und Luft solange wie möglich anhalten". Ebenso kann die Atemanhaltung oder Atemunterbrechung im ausgeatmeten Zustand überprüft werden. In Fig. 2 ist im unteren Abschnitt beispielsweise ein zeitlicher Verlauf 26 dargestellt, wobei ein Plateau 27 mit einer Zeitdauer ΔT erkennbar ist, in der die Untersuchungsperson im eingeatmeten Zustand die Luft angehalten hat. Dieses Plateau kann beispielsweise detektiert werden, indem Zeitabschnitte Δt1, At2 verwendet werden, wobei in diesen Zeitabschnitten die detektierten Signale miteinander verglichen werden. Wenn die Signaländerung von einem Zeitabschnitt zum nächsten Zeitabschnitt geringer als ein Grenzwert ist, so kann die Existenz eines Plateaus abgeschätzt werden. Im dargestellten Beispiel sind die Zeitabschnitte At1 und At2 konsekutive Zeitabschnitte ohne Überlappung. Selbstverständlich können diese Zeitabschnitte At1 und At2 auch überlappen, oder es sind andere Möglichkeiten zur Bestimmung des Plateaus wie die Bildung einer zeitlichen Ableitung möglich, wobei dann ein Plateau identifiziert wird, wenn der Wert der Ableitung nahe 0 liegt. Sobald die Untersuchungsperson nicht mehr die Luft anhalten kann, am Ende des Plateaus, wird dies detektiert, so dass dann die individuelle Dauer der Atemunterbrechung ΔT bestimmt werden kann. Diese gewonnene Information fließt dann automatisch in die Einstellung der Untersuchung ein. Wenn die Untersuchungsperson beispielsweise 15 sec die Luft anhalten kann, wird dieser Wert direkt in die Steuereinheit 20 übertragen, und dort kann beispielsweise die Bildsequenzsteuerung derart angepasst werden, bzw. bei der Bildgebungssequenz verwendeten Bildgebungsparameter können derart angepasst werden, dass ein gewünschtes Untersuchungsvolumen in der Zeitdauer der Atemunterbrechung aufgenommen wird. Ist die Untersuchungsperson nicht in der Lage, die Atmung länger als einen unteren Grenzwert anzuhalten, wobei dieser Grenzwert irgendwo zwischen 5-10 sec liegen kann, so wird eine Bildgebungssequenz gewählt, die mit freier Atmung diagnostisch verwertbare Bilder liefert. Beispielsweise kann dann eine Bildgebungstechnik verwendet werden, die unter dem Namen "Grasp Vibe", "Star WVibe" oder "T2 Blade" bekannt sind.

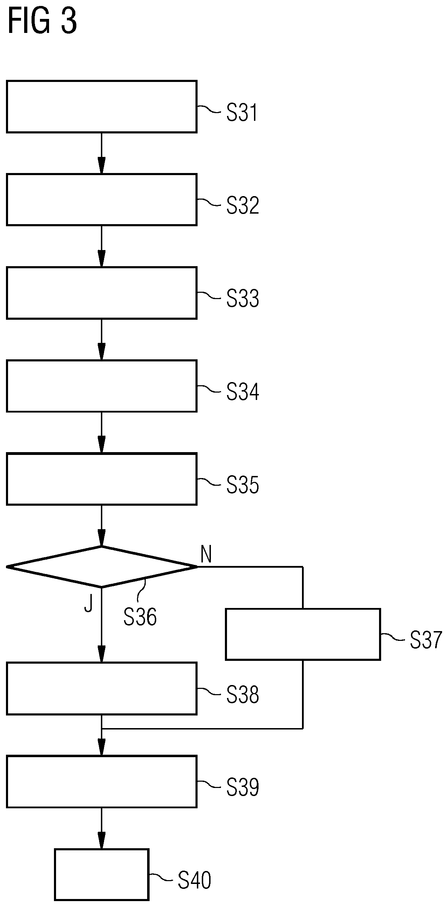

Fig. 3 zeigt ein Flussdiagramm mit einigen Schritten des erfindungsgemäßen Verfahrens. Das Verfahren startet in Schritt S31 und im Schritt S32 wird die Untersuchungsperson auf der Liege positioniert, wobei damit automatisch der in der Liege oder der Spule integrierte Atemsensor in Schritt S33 die Atembewegung detektieren kann. Wenn nun die Untersuchungsperson in der endgültigen Lage in der MR-Anlage liegt, kann das automatische Atemkommando in Schritt S34 abgegeben werden mit der Aufforderung, die Luft solange wie möglich anzuhalten. In Schritt S35 erfolgt dann die Auswertung der Atemkurve, wie sie in Fig. 2 diskutiert wurde. Es wird die Dauer der Atemunterbrechung ΔT bestimmt. Im Schritt S36 wird dann überprüft, ob eine Anpassung der Bildgebungssequenz derart möglich ist, dass die MR-Bilder in der Atemanhalte-Technik aufgenommen werden. Ist dies nicht der Fall, wird in Schritt S37 eine Bildgebungssequenz ausgewählt, die ohne Atemanhalte-Technik noch diagnostisch verwertbare Bilddaten liefert. Hat die Auswertung der Daten in Schritt S36 jedoch ergeben, dass der Patient in der Lage ist, die Atmung lange genug anzuhalten, so werden im Schritt S38 die MR-Bildgebungsparameter an die detektierte Dauer der Atemunterbrechung angepasst. Beispielsweise wird das Gesichtsfeld, die Auflösung, die Anzahl der zu messenden Schichten, etc. so ausgewählt, dass die Aufnahme der MR-Signale innerhalb der Atemunterbrechung möglich ist. Hierbei ist es auch möglich, die gesamten MR-Rohdaten in mehreren Segmenten aufzunehmen, wobei die Untersuchungsperson hierfür die Atmung mehrmals unterbrechen muss. Nach Auswahl der Bildgebungsparameter in Schritt S38 erfolgt schließlich in Schritt S39 die Durchführung der Bildgebungssequenz mit der ausgewählten Bildgebungssequenz und den ausgewählten Bildgebungsparametern. Das Verfahren endet in Schritt S40.

Das oben beschriebene Verfahren erhöht die Reproduzierbarkeit von diagnostisch wertvollen MR-Bildern in Bereichen, die von der Atmung der Untersuchungsperson betroffen sind, d.h. im Abdomen, wie beispielsweise im Oberbauch bei der Aufnahme von MR-Bildern von Organen wie der Leber, der Milz, der Bauchspeicheldrüse, etc. Die Untersuchungen, die aufgrund der nicht möglichen Atemunterbrechung wiederholt werden müssen, werden verringert und minimiert. Weiterhin wird die Bildgebungssequenz an die individuelle Atemanhalte-Fähigkeit der Untersuchungsperson angepasst.

## Patentansprüche

1. Verfahren zur Aufnahme von MR-Bildern einer Untersuchungsperson 13 in einer MR-Anlage 10 mit den folgenden automatisch ablaufenden Schritten:
- Detektieren einer Atembewegung der Untersuchungsperson durch einen an der MR-Anlage angebrachten Atemsensor 15,
- Detektieren einer Atemunterbrechung ΔT bei der Untersuchungsperson und einer Dauer der Atemunterbrechung anhand der detektieren Atembewegung,
- Auswählen einer Bildgebungssequenz zur Erstellung der MR-Bilder unter Berücksichtigung der detektierten Dauer der Atemunterbrechung.

2. Verfahren nach Anspruch 1, **dadurch gekennzeichnet, dass** eine Bildgebungssequenz ausgewählt wird, für die eine Atemunterbrechung mit einer ersten Dauer notwendig ist, wenn die detektierte Dauer der Atemunterbrechung ΔT größer oder gleich der ersten Dauer ist.

3. Verfahren nach Anspruch 1 oder 2, **dadurch gekennzeichnet, dass** eine Bildgebungssequenz ausgewählt wird, die ohne Atemunterbrechung aufgenommen werden kann, wenn die detektierte Dauer der Atemunterbrechung ΔT kleiner als ein vorbestimmter unterer Grenzwert ist.

4. Verfahren nach einem der vorstehenden Ansprüche, **dadurch gekennzeichnet, dass** eine Atemunterbrechung detektiert wird, wenn in einem zeitlichen Verlauf der detektieren Atembewegung ein Plateau 27 detektiert wird, wobei die Dauer der Atemunterbrechung anhand der Länge des Plateaus im zeitlichen Verlauf bestimmt wird.

5. Verfahren nach einem der vorstehenden Ansprüche, **dadurch gekennzeichnet, dass** die Atembewegung von einem Atemsensor 15 detektiert wird, der in eine MR-Empfangsspule 14 zum Empfang von MR-Signalen oder in einer Liege 12 zur Lagerung der Untersuchungsperson integriert ist.

6. Verfahren nach einem der vorstehenden Ansprüche, **dadurch gekennzeichnet, dass** detektiert wird, ob die Untersuchungsperson 13 in der MR-Anlage 10 in einer Aufnahmeposition liegt, bei der die MR-Bilder aufgenommen werden, wobei, wenn dies der Fall ist, ein in einer Sprachdatei gespeichertes Sprachkommando ausgegeben wird, in der die Untersuchungsperson aufgefordert wird, die Luft anzuhalten.

7. Verfahren nach einem der vorstehenden Ansprüche, **dadurch gekennzeichnet, dass** zumindest ein Bildgebungsparameter der Bildgebungssequenz derart angepasst wird, dass die für die Bildgebungssequenz notwendige Atemunterbrechung ΔT kleiner als die detektierte Dauer der Atemunterbrechung ist.

8. Verfahren nach einem der Ansprüche 4 bis 7, **dadurch gekennzeichnet, dass** Zeitabschnitte At1, At2 eines Ausgangssignals des Atemsensors 15 miteinander verglichen werden, wobei in dem zeitlichen Verlauf ein Plateau 27 detektiert wird, wenn eine Änderung des Ausgangssignals in konsekutiven Zeitabschnitten kleiner als ein Grenzwert ist.

9. MR-Anlage 10, die ausgebildet ist für eine Aufnahme von MR-Bildern einer Untersuchungsperson 13, die aufweist:
- einen Atemsensor 15, der ausgebildet ist, automatisch eine Atembewegung der Untersuchungsperson zu detektieren,
- eine Steuereinheit 20, die ausgebildet ist, eine Atemunterbrechung bei der Untersuchungsperson und einer Dauer der Atemunterbrechung anhand der detektieren Atembewegung zu detektieren, und eine Bildgebungssequenz zur Erstellung der MR-Bilder unter Berücksichtigung der detektieren Dauer der Atemunterbrechung auszuwählen.

10. MR-Anlage nach Anspruch 9, **dadurch gekennzeichnet, dass** die Steuereinheit 20 ausgebildet ist, eine Bildgebungssequenz auszuwählen, für die eine Atemunterbrechung mit einer ersten Dauer notwendig ist, wenn die detektierte Dauer der Atemunterbrechung ΔT größer oder gleich der ersten Dauer ist.

11. MR-Anlage nach Anspruch 9 oder 10, **dadurch gekennzeichnet, dass** die Steuereinheit 20 ausgebildet ist, eine Bildgebungssequenz auszuwählen, die ohne Atemunterbrechung aufgenommen werden kann, wenn die detektierte Dauer der Atemunterbrechung ΔT kleiner als ein unterer Grenzwert ist.

12. MR-Anlage nach einem der Ansprüche 9 bis 11, **dadurch gekennzeichnet, dass** die Steuereinheit 20 ausgebildet ist, eine Atemunterbrechung zu detektieren, wenn in einem zeitlichen Verlauf der detektieren Atembewegung ein Plateau 27 detektiert wird, und ausgebildet ist, die Dauer der Atemunterbrechung anhand der Länge des Plateaus im zeitlichen Verlauf zu bestimmen.

13. MR-Anlage nach einem der Ansprüche 9 bis 12, **dadurch gekennzeichnet, dass** die MR-Anlage 10 eine Empfangsspule 14 für den Empfang von MR-Signalen aufweist, in die der Atemsensor 15 integriert ist.

14. MR-Anlage nach Anspruch 13, **dadurch gekennzeichnet, dass** die Empfangsspule 14 eine MR-Rückenspule ist.

15. MR-Anlage nach einem der Ansprüche 9 bis 14, **dadurch gekennzeichnet, dass** die Steuereinheit 20 ausgebildet ist, zumindest ein Bildgebungsparameter der Bildgebungssequenz derart anzupassen, dass die für die Bildgebungssequenz notwendige Atemunterbrechung kleiner als die detektierte Dauer der Atemunterbrechung ist.

16. Computerprogrammprodukt, welches Programmmittel umfasst und direkt in eine Speichereinheit einer programmierbaren Steuereinheit einer MR-Anlage ladbar ist, um alle Schritte des Verfahrens nach einem der Ansprüche 1-8 auszuführen, wenn die Programmmittel in der Steuereinheit ausgeführt werden.

17. Elektronisch lesbarer Datenträger mit darauf gespeicherten elektronisch lesbaren Steuerinformationen, welche derart ausgestaltet sind, dass sie bei Verwendung des Datenträgers in einer Steuereinheit einer MR-Anlage das Verfahren nach einem der Ansprüche 1-8 durchführen.
